# EUROPÄISCHE PATENTSCHRIFT

(11) **EP 3 337 762 B1**
(45) Veröffentlichungstag und Bekanntmachung des Hinweises auf die Patenterteilung: **14.12.2022**
(21) Anmeldenummer: 16748117.5
(22) Anmeldetag: 05.08.2016
(51) Int. Cl.: C01G 49/02, C09C 1/24, A61K 49/06, H01F 1/00

(54) **VERFAHREN ZUR HERSTELLUNG VON STABIL DISPERGIERBAREN MAGNETISCHEN EISENOXID-EINKERN-NANOPARTIKELN, STABIL DISPERGIERBARE MAGNETISCHE EISENOXID-EINKERN-NANOPARTIKEL UND VERWENDUNGEN HIERVON**
METHOD FOR PRODUCING STABLE DISPERSIBLE MAGNETIC IRON OXIDE SINGLE-CORE NANOPARTICLES, STABLE DISPERSIBLE MAGNETIC IRON OXIDE SINGLE-CORE NANOPARTICLES AND USES OF SAME
PROCÉDÉ PERMETTANT DE FABRIQUER DES NANOPARTICULES D'OXYDE DE FER À NOYAU UNIQUE MAGNÉTIQUES ET DISPERSIBLES DE MANIÈRE STABLE, NANOPARTICULES D'OXYDE DE FER À NOYAU UNIQUE MAGNÉTIQUES ET DISPERSIBLES DE MANIÈRE STABLE ET APPLICATIONS DE CELLES-CI

(30) Priorität: 18.08.2015 DE 102015215736
(43) Veröffentlichungstag der Anmeldung: 27.06.2018
(73) Patentinhaber: Fraunhofer-Gesellschaft zur Förderung der angewandten Forschung e.V., 80686 München (DE)
(72) Erfinder: BLEUL, Regina, 65207 Wiesbaden (DE); THIERMANN, Raphael, 65207 Wiesbaden (DE)
(74) Vertreter: Pfenning, Meinig & Partner mbB
(86) Internationale Anmeldenummer: PCT/EP2016/068756
(87) Internationale Veröffentlichungsnummer: WO 2017/029130

(56) Entgegenhaltungen:
- US-A- 4 376 714
- US-A1- 2012 277 517
- MATTHIAS GIROD ET AL: "How temperature determines formation of maghemite nanoparticles", JOURNAL OF MAGNETISM AND MAGNETIC MATERIALS., Bd. 380, 1. Oktober 2014 (2014-10-01), Seiten 163-167, XP055308373, NL ISSN: 0304-8853, DOI: 10.1016/j.jmmm.2014.09.057
- KIWAMU SUE ET AL: "Continuous hydrothermal synthesis of FeO, NiO, and CuO nanoparticles by superrapid heating using a T-type micro mixer at 673K and 30MPa", CHEMICAL ENGINEERING JOURNAL, ELSEVIER SEQUOIA, LAUSANNE, CH, Bd. 166, Nr. 3, 22. November 2010 (2010-11-22), Seiten 947-953, XP028136505, ISSN: 1385-8947, DOI: 10.1016/J.CEJ.2010.11.080 [gefunden am 2010-11-30]
- KIWAMU SUE ET AL: "Continuous Hydrothermal Synthesis of Fe 2 O 3 Nanoparticles Using a Central Collision-Type Micromixer for Rapid and Homogeneous Nucleation at 673 K and 30 MPa", INDUSTRIAL & ENGINEERING CHEMISTRY RESEARCH., Bd. 49, Nr. 18, 15. September 2010 (2010-09-15), Seiten 8841-8846, XP055308315, US ISSN: 0888-5885, DOI: 10.1021/ie1008597

## Beschreibung

Die vorliegende Erfindung betrifft magnetische Einkern-Nanopartikel, insbesondere stabil dispergierbare magnetische Einkern-Nanopartikel (z.B. Einkern-Magnetit-Nanopartikel) mit einem Durchmesser zwischen 20 und 200 nm in unterschiedlicher Morphologie und deren kontinuierliche, wässrige Synthese, insbesondere mit Hilfe von Mikromischern. Das Verfahren ist einfach, schnell und kostengünstig durchführbar und kommt ohne organische Lösungsmittel aus. Die durch das Verfahren hergestellten Einkern-Nanopartikel bilden in wässrigen Medien stabile Dispersionen aus d.h. sie neigen nicht zur Assemblierung bzw. Aggregation. Darüberhinaus bietet das Verfahren die Möglichkeit, anisotrope, superparamagnetische, plättchenförmige Nanopartikel herzustellen, die sich aufgrund ihrer Formanisotropie hervorragend für die Verwendung in polymeren Matrices zur Magnetfeld-gesteuerten Freisetzung von Wirkstoffen eignen.

Es gibt derzeit keine Möglichkeit, auf eine wirtschaftliche Weise einzeln stabilisierte Einkern-Eisenoxidnanopartikel herzustellen, deren Durchmesser größer als 20 nm ist. Es gibt jedoch verschiedene Anwendungen, für welche insbesondere nanopartikuläre magnetische Einkern-Eisenoxide, welche größer als 20 nm im Durchmesser sind, einzeln stabilisiert im Dispersionsmedium vorliegen müssen, d.h. keine Aggregation der Nanopartikel erfolgen darf. Bei diesen Anwendungen handelt es sich z.B. um die Magnetfluid-Hyperthermie, die magnetische Separation, den magnetischen Wirkstofftransport, die magnetische Wirkstofffreisetzung, Immunoassays, diagnostische Anwendungen und das sog. "magnetic particle imaging".

Eine Herstellungsmethode von magnetischen Eisenoxid-Nanopartikeln ist die Standardmethode der Co-Präzipitation nach dem Verfahren von Massart (siehe DE 10 2008 015365 A1) welche auch zur industriellen Herstellung von Ferrofluiden genutzt wird. Diese liefert jedoch keine Einkern-Eisenoxid-Nanopartikel, die eine Größe von mindestens 20 nm aufweisen. Daher ist sie ungeeignet zur Bereitstellung von Nanopartikeln mit einer Größe von 20 bis 200 nm.

Gängige Herstellungsmethoden von magnetischen Eisenoxid-Einkern-Partikeln mit einer Größe von mindestens 20 nm basieren auf der thermischen Zersetzung organischer Vorläufermoleküle in hochsiedenden organischen Lösungsmitteln. Diese Verfahren sind sehr energieaufwändig und verbrauchen große Mengen an organischen Lösungsmitteln (siehe z.B. US 2006/0133990 A1). Erstens lassen sich diese Methoden schlecht in den industriellen Maßstab überführen. Zweitens weisen die in siedenden organischen Lösungsmitteln hergestellten Nanopartikel hydrophobe Eigenschaften auf. Folglich ist ihre Überführung in ein wässriges Medium von der Bildung von Nanopartikel-Aggregaten begleitet (hydrophober Effekt), was ein bisher nur unzureichend gelöstes Problem darstellt.

Es ist bekannt, dass Eisenoxid-Nanopartikel mit einer Größe von mehr als 50 nm und hydrophilen Eigenschaften durch die Oxidationsmethode von Sugimoto hergestellt werden können. Hierbei wird zunächst Fe(OH)₂ aus einer Fe(II)-Lösung in alkalischem Medium ausgefällt und anschließend durch ein zugesetztes Oxidationsmittel unter Temperatureinwirkung zu Magnetit oxidiert (Sugimoto & Matijevic, J. Colloid Interface Sci. 1980, 74 (1), 227-243). Die auf diese Weise hergestellten Partikel weisen aufgrund ihrer Größe jedoch bereits ferrimagnetische Eigenschaften auf und neigen zudem zur Aggregation.

Zusammenfassend ist festzuhalten, dass weder mit der Methode nach Massart noch mit der Oxidationsmethode von Sugimoto einzeln stabilisierte Einkern-Eisenoxid-Nanopartikel in der Größe zwischen 20 und 200 nm zugänglich sind, welche superparamagnetische bis ferrimagnetische Eigenschaften und eine geringe Aggregationsanfälligkeit aufweisen und somit auch für anspruchsvolle medizinische Anwendungen (z.B. Hyperthermie oder "magnetic particle imaging") geeignet sind.

Die bislang einzig bekannte Möglichkeit, in wässrigen Medien stabil dispergierbare Einkern-Eisenoxid-Nanopartikel in diesem Größenbereich herzustellen ist eine Herstellung auf biotechnologischem Weg mit Hilfe von Mikroorganismen (Lang et al. Macromol. Biosci. 2007, 7, 144-151). Die dadurch hergestellten Einkern-Eisenoxid-Nanopartikel werden als Magnetosomen bezeichnet und weisen tatsächlich sehr gute physikalische Eigenschaften (z.B. für das "magnetic particle imaging") auf. Die Magnetosomen haben jedoch den entscheidenden Nachteil, dass sie aufgrund ihres bakteriellen Ursprungs noch bakterielle Antigene enthalten und somit stark immunogen sind. Für eine medizinische Anwendung sind sie somit ungeeignet. Außerdem können sie bislang nur sehr aufwändig und in geringen Mengen hergestellt werden.

Ausgehend hiervon war es die Aufgabe der vorliegenden Erfindung eine Verfahren zur Herstellung von Nanopartikeln bereitzustellen, die nicht immunogen sind, bevorzugt superparamagnetische Eigenschaften und ein vergleichsweise hohes magnetisches Moment aufweisen und nicht zur Aggregationsbildung in wässrigen Medien neigen.

Die Aufgabe wird gelöst durch das Verfahren gemäß Anspruch 1, die magnetischen Einkern-Nanopartikel gemäß Anspruch 11 und die Verwendung der magnetischen Einkern-Nanopartikel gemäß Anspruch 15.

Erfindungsgemäß wird ein Verfahren zur kontinuierlichen Herstellung von stabil dispergierbaren magnetischen Einkern-Nanopartikeln, die in wässrigen Medien nicht zur Aggregation neigen, die Eisenoxid und Stabilisator enthalten und einen Durchmesser zwischen 20 und 200 nm aufweisen, bereitgestellt. Das Verfahren umfasst die Schritte
a) Bereitstellen einer wässrigen Lösung enthaltend mindestens eine Base und mindestens ein Oxidationsmittel;
b) Bereitstellen einer wässrigen Lösung enthaltend mindestens ein Eisensalz, bevorzugt mindestens ein Fe(II)-Salz, besonders bevorzugt mindestens ein Fe(II)-Salz und ein von einem Fe(II)-Salz verschiedenes Eisensalz in einer niedrigeren Konzentration als das Fe(II)-Salz, wobei die Konzentration von Fe(II)-Salz zu dem von dem Fe(II)-Salz verschiedenen Eisensalz insbesondere 4:1 oder größer ist (optional ist in dieser wässrigen Lösung kein Fe(III)-Salz enthalten);
c) Bereitstellen einer wässrigen Lösung enthaltend mindestens einen hydrophilen Stabilisator, wobei der hydrophile Stabilisator eine Substanz enthält oder daraus besteht, die ausgewählt ist aus der Gruppe bestehend aus glykosidische Flavonoide, Phenole, Phosphorsäurederivate, Triphosphat, Polymere mit mehr als 3 Histidin-Resten, hydrophile Polymere, und Carbonsäure oder Carbonsäureanhydrid;
d) Mischen der wässrigen Lösung aus a) und b) zu einem Gemisch in einem Mikromischer, wobei Fe(OH)₂ gebildet wird, das aus der Lösung ausfällt und zu magnetischen Einkern-Nanopartikeln oxidiert; und
e) Mischen des Gemisches aus d) mit der wässrigen Lösung aus c), wobei der mindestens eine Stabilisator an das Eisenoxid bindet;

Das Verfahren ist dadurch gekennzeichnet, dass in a) bis e) auf eine Temperatur von 10°C bis 200 °C temperiert wird, wobei in Schritt e) stabil dispergierbare magnetische Einkern-Nanopartikel entstehen, die in wässrigen Medien nicht zur Aggregation neigen, die Eisenoxid und den Stabilisator enthalten und einen Durchmesser zwischen 20 und 200 nm aufweisen.

Unter Nanopartikeln werden erfindungsgemäß Partikel verstanden, die verschiedene Morphologien aufweisen können (z.B. kugelförmig, ellipsoid, quaderförmig oder scheibenförmig) und einen Durchmesser von 20 bis 200 nm aufweisen. Unter Durchmesser wird die längste dreidimensionale Ausdehnung der Partikel verstanden (z.B. bei Kugeln der Kugeldurchmesser und bei Scheiben der Scheibendurchmesser). Unter Einkern-Nanopartikel wird verstanden, dass es sich bei den Nanopartikeln nicht um Cluster mehrerer Nanopartikel (Mehrkern-Nanopartikel), sondern um einzelne Nanopartikel (Einzeldomänen-Nanokristalle) handelt.

Der Kernaspekt der Erfindung ist die Verwendung eines Stabilisators bei der Herstellung der Nanopartikel, der sowohl an Eisenoxid (d.h. den sich während des Verfahrens bildenden Eisenoxidkern) binden kann als auch dessen Stabilisierung (Ausbildung einer homogenen Dispersion) in Wasser vermittelt. Durch die Zugabe des Stabilisators in Schritt e) wird eine weitere Anlagerung von Eisenoxid an die in Schritt d) gebildeten Eisenoxid-Nanopartikel gehemmt d.h. das weitere Wachstum der Eisenoxid-Nanopartikel wird durch den Stabilisator unterdrückt. Folglich kann durch eine zeitliche Variation der Zugabe des Stabilisators nach Schritt d) des erfindungsgemäßen Verfahrens die Größe der Nanopartikel beeinflusst werden. Je nach Konzentration der Edukte lassen sich somit in einem Zeitintervall von 1 Sekunde bis 24 Stunden unproblematisch Nanopartikel bereitstellen, die einen Durchmesser von 20 bis 200 nm aufweisen. Es hat sich herausgestellt, dass bei geeigneter Wahl der Temperatur und der Konzentrationen der Ausgangslösungen sogar eine Bereitstellung erfindungsgemäßer Nanopartikel in einem Zeitrahmen von weniger als 10 Minuten möglich ist. Insofern sind in dem erfindungsgemäßen Verfahren sehr kurze Inkubationszeiten möglich. Es wurde zudem gefunden, dass sich durch die zeitliche Variation (z.B. im Zeitraum von 1 sek. bis unter 10 Minuten) nicht nur die Größe, sondern auch die Morphologie der Nanopartikel einstellen lässt, wobei kürzere Zeitintervalle eine flache Form (Plättchenform) und längere Zeitintervalle eine rundere Form (Sphärenform bzw. Quaderform) bewirken.

Mit dem erfindungsgemäßen Verfahren können somit auch anisotrope plättchenförmige magnetische Einkern-Eisenoxid-Nanopartikel hergestellt werden, die beispielsweise eine Dimension von ca. 30 nm Durchmesser und 3 nm Dicke aufweisen. Durch die Plättchenform-bedingte Anisotropie (Formanisotropie) und die superparamagnetischen Eigenschaften sind diese Einkern-Nanopartikel für besondere Anwendungen geeignet. Unter Einwirkung eines magnetischen Feldes richten sich die parallelen magnetischen Feldlinien entlang des magnetischen Feldes aus und bewirken eine Bewegungskraft auf die plättchenförmigen Nanopartikel. Alterniert das magnetische Feld, so wird das Plättchen in der Frequenz der Alternation hin und her bewegt. Durch diese Bewegung ist es beispielsweise möglich, über die anisotropen magnetischen Nanopartikel die Freisetzung eines Wirkstoffes aus einer Polymermatrix zu steuern und/oder Wärme zu erzeugen. Folglich eignen sich die erfindungsgemäßen Nanopartikel ausgezeichnet für die Verwendung in polymeren Matrizes zur Magnetfeld-gesteuerten Freisetzung von Wirkstoffen und/oder für Hyperthermie-Anwendungen.

Das erfindungsgemäße Verfahren ist einfach und kostengünstig durchführbar, da die Synthese und Stabilisierung inklusive einer darauf folgenden Aufreinigung kontinuierlich im Durchfluss durchgeführt werden kann. Dies erlaubt sehr kurze Synthesezeiten (z.T. wenige Minuten). Ein weiterer Vorteil ist, dass die Synthese in einem wässrigen System erfolgt, was den Bedarf an organischen Lösungsmitteln obsolet macht und die Durchführung des Verfahrens umweltfreundlicher gestaltet. Zusätzliche, z.B. bei der Synthese mit organischen Lösungsmitteln oder der Co-Präzipitation nach dem Verfahren von Massart notwendige Verfahrensschritte (z.B. Fällen und Resuspendieren), sind in dem erfindungsgemäßen Verfahren nicht nötig, was weiterhin zur Verkürzung und Vereinfachung der Synthese beiträgt. Darüberhinaus ist ein entscheidender Vorteil des erfindungsgemäßen Verfahrens, dass die bereitgestellten magnetischen Einkern-Nanopartikel in wässrigen Medien nicht zur Assemblierung bzw. Aggregation ("Clusterbildung") neigen, also in wässrigen Medien homogene, stabile Dispersionen bilden.

Das erfindungsgemäße Verfahren kann dadurch gekennzeichnet sein, dass in Schritt d), optional auch in Schritt e), mit einem Mikromischer gemäß DIN EN ISO 10991:2010-03 gemischt wird. Die Verwendung eines Mikromischers hat diverse Vorteile gegenüber einer Mischung im Batchverfahren. Durch den Mikromischer wird eine kontinuierliche, reproduzierbare Herstellung und Stabilisierung der Einkern-Nanopartikel erreicht und kann durch eine sich anschließende ggf. ebenfalls kontinuierlich verlaufende Aufreinigung (z.B. über Ultrafiltration und/oder Diafiltration) ergänzt werden. Ein Mischen mit einem Magnetrührer ist nicht nötig, wodurch die Bildung von Agglomeraten vermieden wird. Darüberhinaus ermöglicht die Verwendung eines Mikromischers die Durchführung des Verfahrens in einem geschlossenen System d.h. beispielsweise eine Verfahrensführung in Abwesenheit von Luftsauerstoff. Zuvor bereitgestellte entgaste Eduktlösungen bleiben somit in diesem entgasten Zustand.

Der Mikromischer kann einen Multilaminations-Mikromischer, turbulenten Mikromischer, "impinging jet"-Mikromischer und/oder "split-and-recombine"-Mikromischer enthalten oder daraus bestehen, wobei der Mikromischer optional im Anschluss an den Mischbereich oder Mischraum durch einen sich im Wesentlichen nicht verengenden und/oder im Wesentlichen geraden Ausgang ohne abrupte Richtungsänderung und/oder Querschnittsverengung des Fluidstroms gekennzeichnet ist. Insbesondere bevorzugt sind "caterpillar"-Mikromischer, bspw. ein Mikromischer mit der Bezeichnung "CPMM R300x12-SO" (entwickelt von Fraunhofer ICT IMM vormals Institut für Mikrotechnik Mainz GmbH, IMM) und/oder "slit interdigital"-Mikromischer. Die genannten sowie auch weitere nutzbare Mikromischer sind in der Veröffentlichung von Hessel V. et al. in Chemical Micro. Process. Engineering: Processing and Plants, Wiley 2005 offenbart. Die Mikromischer können aus Metallen oder Legierungen, vorzugsweise Edelstahl, Keramik und/oder Kunstoffen, bevorzugt Polyetheretherketon, hergestellt werden. Polyetheretherketon hat den Vorteil, dass es sehr widerstandsfähig ist.

Der hydrophile Stabilisator kann mindestens einen an Eisenoxid bindenden Rest, bevorzugt mindestens zwei an Eisenoxid bindende Reste aufweisen. Die Reste binden vorzugswiese über eine koordinative Bindung an das Eisenoxid. Der oder die Reste können an ein hydrophiles Polymer geknüpft sein, wobei die Reste bevorzugt ausgewählt sind aus der Gruppe bestehend aus Phenol-Rest, PO₃-Rest, Histidin-Rest und Zucker-Rest. Optional ist der Rest bzw. sind die Reste ausgewählt aus der Gruppe bestehend aus Phenol-Rest, PO₃-Rest und Histidin-Rest. Bei dem hydrophilen Polymer handelt es sich bevorzugt um PEG oder ein PEG-Derivat. Die (chemisch kovalente) Verknüpfung von PEG bzw. einem PEG-Derivat mit einer Substanz, die einen der oben genannten Reste aufweist stellen besonders bevorzugte Stabilisatoren dar.

Der Stabilisator enthält erfindungsgemäß eine Substanz ausgewählt aus der Gruppe bestehend aus
a) glykosidische Flavonoide, bevorzugt Rutin und/oder Hesperidin, besonders bevorzugt Rutinhydrat;
b) Phenole, bevorzugt Polyphenole, weiter bevorzugt Quercetin, Tannin, Catechol und/oder Lignin, besonders bevorzugt Ligninsulfonat und/oder Catechol-PEG;
c) Phosphorsäurederivate, bevorzugt Bisphonat, besonders bevorzugt Alendronsäure und/oder Derivate hiervon;
d) Triphosphat, bevorzugt Pentanatriumtriphosphat und/oder Pentakaliumtriphosphat;
e) Polymere mit mehr als 3 Histidin-Resten;
f) hydrophile Polymere, bevorzugt Stärke, Chitosan, Dextran; und
g) Carbonsäure oder Carbonsäureanhydrid, bevorzugt Polymaleinsäureanhydrid und Derivate hiervon;
oder besteht daraus;
bevorzugt glykosidische Flavonoide und/oder hydrophile Polymere, besonders bevorzugt ein kovalent mit einem hydrophilen Polymer verknüpftes glykosidisches Flavonoid, insbesondere Rutin-PEG und/oder Catechol-PEG. Generell ist vorteilhaft, wenn die oben genannte Substanz (chemisch kovalent) mit PEG bzw. einem PEG-Derivat verknüpft ist. In einer bevorzugten Ausführungsform wird Rutinhydrat verwendet, da es sehr gut auch in Anwesenheit der anderen Agenzien löslich ist.

Die wässrige Lösung in a), b), und/oder c), bevorzugt a) bis c), wird bevorzugt in demineralisiertem und/oder entgastem Wasser bereitgestellt. Die Verwendung von demineralisiertem Wasser hat den Vorteil, dass keine unerwünschten Ionen die Eisenhydroxidbildung und Eisenoxidbildung während des Verfahrens negativ beeinflussen. Die Verwendung von entgastem Wasser hat den Vorteil, dass bei der Mischung in den Schritten d) und e) Oxidationseffekte durch Luftsauerstoff und die Bildung von Luftbläschen mit unerwünschten Grenzflächeneffekten verhindert werden. Bei der Entgasung werden mindestens 50 %, vorzugweise mehr als 75%, des im Gleichgewicht bei 20 °C gelösten Sauerstoffs entfernt.

In Schritt a), b), c), d) und/oder e) bevorzugt in den Schritten a) bis e), wird bevorzugt auf eine Temperatur von 10 °C bis 200 °C, bevorzugt 40 bis 100 °C, besonders bevorzugt 50 bis 70 °C, insbesondere 60 °C, temperiert. Die Durchführung des Verfahrens in diesem Temperaturbereich hat sich als besonders vorteilhaft für die schnelle und selektive Bildung von Eisenoxid-Einkern-Nanopartikeln mit einem Durchmesser von 20 bis 200 nm herausgestellt. Es wurde zudem beobachtet, dass über die Einstellung der Temperatur (z.B. im Rahmen von 40 °C bis 80 °C) eine Feinjustierung der Größe der erzeugten Nanopartikel möglich ist. Hierbei wurde der Zusammenhang gefunden, dass eine Temperierung auf höhere Temperaturen bei sonst gleichen Bedingungen einen leicht größeren Durchmesser der hergestellten magnetischen Einkern-Nanopartikeln bewirkt.

Bevorzugt wird vor und/oder nach Zugabe des Stabilisators für einen Zeitraum von 1 sek. bis 24 h, bevorzugt 10 sek. bis 4 h, besonders bevorzugt 30 sek. bis 40 min., insbesondere 1 min. bis 20 min., inkubiert. Die Inkubation erfolgt insbesondere in einer temperierten Verweilschleife. Die Verwendung einer temperierten Verweilschleife hat den Vorteil, dass bei einer bestimmten Flussrate durch die Verweilschleife der Zeitpunkt des Austritts der Lösung aus der Verweilschleife exakt definiert ist. Da erfindungsgemäß das Gemisch aus Schritt d) des Verfahrens mit einer wässrigen Lösung mit Stabilisator gemischt wird, legt die temperierte Verweilschleife somit exakt den Zeitpunkt fest, an dem das Gemisch aus Schritt d) mit dem Stabilisator kontaktiert wird und ein weiteres Wachstum der Eisenoxid-Nanopartikel gehemmt wird.

Eine kurze Inkubationszeit vor der Zugabe des Stabilisators favorisiert eine Bildung von Eisenoxid-Einkern-Nanopartikeln, die eine Plättchenform aufweisen, längere Inkubationszeiten begünstigen eine Sphärenform bzw. Quaderform. Am bevorzugtesten erfolgt die Inkubation vor der Zugabe des Stabilisators für einen Zeitraum von weniger als 10 Minuten, da sich herausgestellt hat, dass dieser Zeitraum ausreicht, um Nanopartikel in dem gewünschten Größenbereich in Plättchenform oder Sphärenform bzw. Quaderform über das erfindungsgemäße Verfahren herzustellen. Die kurze Inkubationszeit von weniger als 10 Minuten bewirkt, dass über das erfindungsgemäße Verfahren kontinuierlich eine große Menge stabil dispergierbarer magnetischer Einkern-Nanopartikeln mit der gewünschten Größen und Morphologie pro Zeiteinheit bereitgestellt werden kann. Mit anderen Worten ist mit dem erfindungsgemäßen Verfahren ein hoher Durchsatz möglich.

In einem weiteren Schritt des erfindungsgemäßen Verfahrens kann die Oberfläche der stabilisierten magnetischen Einkern-Nanopartikel modifiziert werden, wobei der hierbei genutzte Modifikator bevorzugt eine Substanz ausgewählt aus der Gruppe bestehend aus
a) anorganische Oxide, bevorzugt Siliciumoxid;
b) anorganische Phosphate, bevorzugt Hydroxyapatit;
c) Hartstoffe, bevorzugt Siliciumcarbid;
d) organische Polymere, bevorzugt PEG, PEG-Silan, Polylactid, Polystyrol, Polymethacrylat, Polyvinylalkohol, Polyvinylpyrrolidon, Polyglycolid und/oder Polycaprolacton; und/oder
e) anorganische Polymere, bevorzugt Polysiloxan;
enthält oder daraus besteht. Insbesondere wird unter der Modifizierung eine Anbindung mindestens eines Modifikators an die Oberfläche der stabilisierten magnetischen Einkern-Nanopartikel über mindestens eine chemisch kovalente Bindung und/oder über mindestens eine nicht-kovalente Wechselwirkung (Wasserstoffbrückenbindung, Dipol-Dipol-Wechselwirkung, ionische Wechselwirkung und/oder hydrophober Effekt) verstanden. Die Modifizierung der Oberfläche (z.B. während oder nach Schritt e)) erweitert die Anwendungsmöglichkeiten der Einkern-Nanopartikel mit einer spezifischen Funktionalisierung der Oberfläche und/oder kann auch deren Langzeitstabilität weiter verbessern.

Die Konzentration
a) der Base kann 10 mM bis 5 M betragen, bevorzugt 15 mM bis 1 M, besonders bevorzugt 20 mM bis 120 mM;
b) des Oxidationsmittels kann 10 mM bis 5 M betragen, bevorzugt 15 mM bis 1 M, besonders bevorzugt 20 mM bis 120 mM; und/oder
c) des Fe(II)-Salzes kann 10 mM bis 5 M betragen, bevorzugt 50 mM bis 500 mM, besonders bevorzugt 100 mM bis 200 mM; und/oder
d) des Stabilisators kann 1 mM bis 5 M betragen, bevorzugt 10 mM bis 1 M, besonders bevorzugt 15 mM bis 120 mM, insbesondere 20 mM bis 50 mM.

Das molare Konzentrationsverhältnis von
a) Fe²⁺-Ionen zu OH⁻-Ionen kann 4:1 bis 1:8, bevorzugt 2:1 bis 1:4 betragen;
b) NO₃⁻-Ionen zu Fe²⁺-Ionen kann 20:1 bis 1:4, bevorzugt 10:1 bis 1:1 betragen; und/oder
c) NO₃⁻-Ionen zu OH⁻-Ionen kann 1:5 bis 10:1, bevorzugt 1:2 bis 5:1, betragen.

Die Base kann ein Alkalihydroxid und/oder Ammoniumhydroxid enthalten oder daraus bestehen, bevorzugt Natriumhydroxid, Kaliumhydroxid, Ammoniumhydroxid und/oder Trimethylammoniumhydroxid.

Das Oxidationsmittel kann ein Nitratsalz und/oder HNO₃ enthalten oder daraus bestehen, bevorzugt ein Alkalinitrat und/oder Ammoniumnitrat, besonders bevorzugt Natriumnitrat, Kaliumnitrat und/oder Ammoniumnitrat. Insbesondere enthält das Oxidationsmittel keine Fe(III)-Ionen, was die anteilige Bildung von Partikeln mit deutlich kleineren Partikeldurchmessern als 20 nm unterdrückt.

Das Fe(II)-Salz kann ein Fe(II)-Halogenid und/oder Fe(II)-Sulfat, bevorzugt Fe(II)chlorid und/oder Fe(II)sulfat, enthalten oder daraus bestehen.

Bevorzugt werden die Einkern-Nanopartikel in einem weiteren Schritt nach Schritt e) aufgereinigt, bevorzugt über Diafiltration und/oder Ultrafiltration, wobei die Einkern-Nanopartikel insbesondere von Salzen und überschüssigem Stabilisator befreit werden.

Ferner werden erfindungsgemäß magnetische Einkern-Nanopartikel mit einem Durchmesser zwischen 20 und 200 nm, enthaltend Eisenoxid und mindestens einen Stabilisator bereitgestellt, die durch das erfindungsgemäße Verfahren herstellbar sind, wobei der hydrophile Stabilisator eine Substanz ausgewählt aus der Gruppe bestehend aus glykosidische Flavonoide, Phenole, Bisphosphonat, Triphosphat und Polymere mit mehr als 3 Histidin-Resten enthält oder daraus besteht. Im Gegensatz zu Einkern-Nanopartikeln bakteriellen Ursprungs weisen die erfindungsgemäßen Einkern-Nanopartikel keine bakteriellen Antigene auf (auch keine Spuren davon), was sie immuntoleranter macht. Die erfindungsgemäßen Einkern-Nanopartikel weisen eine Hülle-Kern-Form auf mit einem Eisenoxid-Einkern und eine den Einkern umgebenden Hülle, die durch den Stabilisator gebildet wird und/oder ihn enthält.

Die magnetischen Einkern-Nanopartikel können auch die Form eines Plättchens aufweisen, bevorzugt ein Plättchen mit einem Durchmesser von 20 bis 200 nm und/oder einer Dicke von 2 bis 20 nm, besonders bevorzugt mit einem Durchmesser von 20 bis 40 nm und/oder einer Dicke von 2 bis 10 nm, ganz besonders bevorzugt mit einer Dicke von 2 nm bis 6 nm. Die plättchenförmigen Einkern-Nanopartikel weisen eine starke Formanisotropie auf und sind superparamagnetisch. Die Formanisotropie ermöglicht im alternierenden magnetischen Feld eine mechanische "Verletzung" ihrer Umgebung (Matrix) und eine Temperaturerhöhung. Außerdem weisen Plättchen mit gleichem Durchmesser zu Vollkugeln ein geringeres Gewicht als die Vollkugeln auf, was generell ihre Dispersionstabilität verbessert.

Stabil-dispergierte superparamagnetische Einkern-Magnetpartikel sind im besonderen Maße für medizinische Anwendungen interessant, da dort die hydrodynamische Größe beispielsweise über die Biodistribution entscheidet. Für ein magnetisches Targeting erleichtert ein hohes magnetisches Moment die Steuerung der Partikel im Körper enorm. Auch für Hyperthermie-Anwendungen sind Einkern-Nanopartikel in diesem Größenbereich von besonderem Interesse, da im Allgemeinen auch die spezifische Heizrate mit der Größe des Magnetkerns zunimmt.

Folglich wird die Verwendung der erfindungsgemäßen magnetischen Einkern-Nanopartikel in der Medizin vorgeschlagen, bevorzugt in einem Verfahren zur chirurgischen oder therapeutischen Behandlung des menschlichen oder tierischen Körpers, besonders bevorzugt
a) zur Magnetfeld-gesteuerten Freisetzung von einem Wirkstoff;
b) zum Magnetfeld-gesteuerten Targeting von einem Wirkstoff;
c) zur Behandlung einer Krankheit mit Magnet-Fluid-Hyperthermie; und/oder
d) als Kontrastmittel oder Tracer in der medizinischen Bildgebung, bevorzugt in der Magnetresonanztomographie und/oder dem "Magnetic Particle Imaging";
in einem Verfahren zur chirurgischen oder therapeutischen Behandlung des menschlichen oder tierischen Körpers, wobei es sich bei der Behandlung insbesondere um die Behandlung von Krebs handelt.

Darüberhinaus wird die Verwendung der erfindungsgemäßen magnetischen Einkern-Nanopartikel in der Diagnostik vorgeschlagen, bevorzugt als Sensor in der Diagnostik, besonders bevorzugt als Sensor in einem Diagnostizierverfahren, das am menschlichen oder tierischen Körper vorgenommen wird.

Ferner finden die erfindungsgemäßen Einkern-Nanopartikel Verwendung zur
a) Magnetfeld-gesteuerten Freisetzung von Substanzen;
b) Magnetfeld-gesteuerten Separation von Substanzen;
c) Magnetfeld-gesteuerten Schaltung elektronischer Komponenten;
d) Verwendung als Kontrastmittel oder Tracer in der Bildgebung, bevorzugt in der Magnetresonanztomographie und/oder dem "Magnetic Particle Imaging";
e) Herstellung von Kompositmaterialien; und/oder
f) Herstellung eines Ferrofluids.

Es wird hiermit klargestellt, dass es sich bei den in diesem Absatz aufgeführten Magnetfeld-gesteuerten Verwendungen a) bis f) nicht um Verwendungen handelt, die ein Verfahren zur chirurgischen oder therapeutischen Behandlung des menschlichen oder tierischen Körpers betreffen.

Anhand der folgenden Figuren und Beispiele soll der erfindungsgemäße Gegenstand näher erläutert werden, ohne diesen auf die hier dargestellten spezifischen Ausführungsformen einschränken zu wollen.

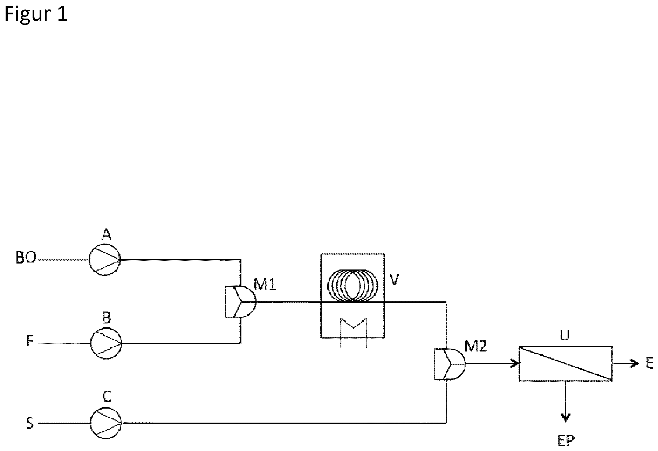

Figur 1 zeigt eine schematische Darstellung eines erfindungsgemäßen Verfahrens. Eine erste wässrige Lösung BO enthaltend eine Base und ein Oxidationsmittel wird über die Pumpe A mit einer Geschwindigkeit von 7 bis 10 ml/min zu dem ersten Mikromischer M1 gepumpt. Dort trifft die erste wässrige Lösung BO auf die zweite wässrige Lösung F, die mindestens ein Fe(II)-Salz enthält und über die Pumpe B mit einer Geschwindigkeit von 1 bis 1,5 ml/min in den Mikromischer M1 gepumpt wird. Im Mikromischer M1 werden die erste und zweite wässrige Lösung BO, F gemischt und das Gemisch in eine temperierte Verweilschleife V gepumpt, die einen Innendurchmesser von ca. 0,5 mm - 1,6 mm und eine bestimmte, vordefinierte Länge aufweist. Die Länge der Verweilschleife V legt die Inkubationszeit fest, bevor das Gemisch mit einer wässrigen Lösung enthaltend Stabilisator kontaktiert wird. Sollen plättchenförmige Nanopartikel hergestellt werden, ist die Länge der Verweilschleife V kurz (z.B. 2 m => kurze Inkubationsdauer). Sollen sphärische Nanopartikel hergestellt werden, ist die Länge der Verweilschleife V lang (z.B. 50 m => lange Inkubationsdauer). Nach Passieren der Verweilschleife wird das Gemisch in den Mikromischer M2 geführt. Dort trifft das Gemisch auf die wässrige Lösung S enthaltend mindestens einen Stabilisator, die in den Mikromischer M2 über die Pumpe C mit einer Geschwindigkeit von 2 bis 3 ml/min gepumpt wird. Im Mikromischer M2 entstehen erfindungsgemäße hydrophil-stabilisierte Eisenoxid-Einkern-Nanopartikel, die über ein Ultrafiltrationsmodul U weiter aufgereinigt werden. Überschüssige Edukte E (Salze und überschüssiger Stabilisator) werden somit von dem Produkt EP (Einkern-Nanopartikel) getrennt.

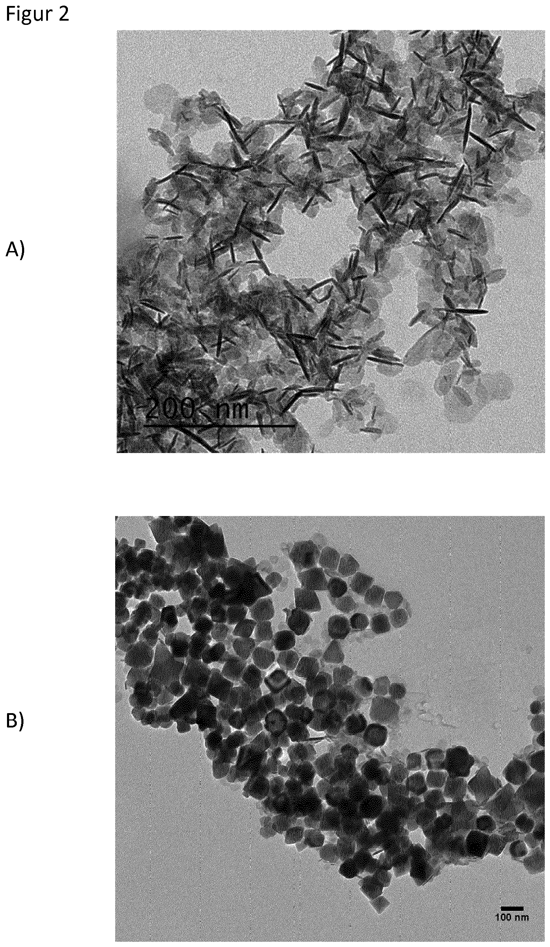

Figur 2 zeigt eine TEM-Aufnahme von erfindungsgemäß hergestellten Nanopartikeln. In Figur 2A sind plättchenförmige Nanopartikel (Durchmesser ca. 30 nm, Dicke ca. 3 nm) dargestellt, die mithilfe einer kurzen Verweilschleife hergestellt wurden. Figur 2B zeigt sphärische bis quaderförmige Nanopartikel (Durchmesser ca. 80 nm), die mithilfe einer langen Verweilschleife hergestellt wurden.

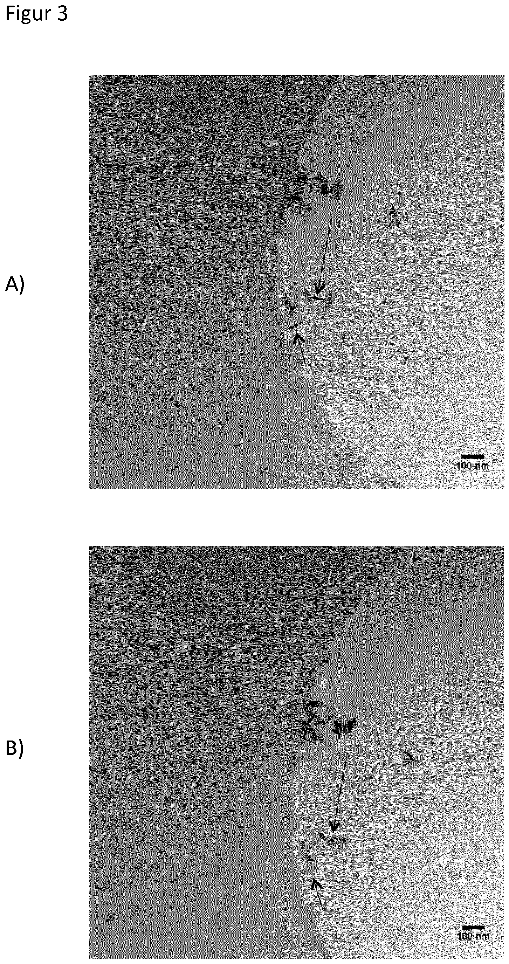

Figur 3 zeigt eine Cryo-TEM-Aufnahme von erfindungsgemäßen plättchenförmigen Nanopartikeln. Die Verkippung der Probe aus Fig. 3B um 30° nach gegenüber der Probe aus Fig. 3A belegt den Scheibencharakter der Nanopartikel (siehe die beiden mit Pfeilen markierten Einkern-Nanopartikel).

### Beispiel 1 - Herstellung von magnetischen Eisenoxid-Einkern-Nanopartikeln in Scheibenform

| | |
|---|---|
| Ausgangslösung BO: | 60 mM NaOH, 54 mM NaNO₃ in entgasten und demineralisierten Wasser; |
| Ausgangslösung F: | 0,1 M FeCl₂ in entgastem Wasser; |
| Stabilisatorlösung S: | 20 mM Rutinhydrat in 60 mM NaOH. |

Alle Reaktionslösungen wurden auf 60 °C vorgeheizt, die Reaktion erfolgte bei 60 °C.

Pumpe A wurde mit 10 ml/min, Pumpe B mit 1,5 ml/min und Pumpe C mit 3 ml/min betrieben. Die Verweilschleife mit Innendurchmesser 0,5 mm war 2 m lang. Verwendet wurde ein Mikromischer mit der Bezeichnung "CPMM R300x12-SO" aus Polyetheretherketon. Die mit diesem Verfahren hergestellten scheibenförmigen Einkern-Nanopartikel sind in Fig. 2A dargestellt.

Besondere Merkmale dieser scheibenförmigen Magnet-Partikel sind neben der Formanisotropie, die eine magnetische Schaltung ermöglicht auch deren besondere magnetische Eigenschaften, was sie besonders für einen Einsatz in der (medizinischen) Bildgebung prädestiniert. So wiesen diese Partikel für ihre potentielle Anwendung im "Magnetic Particle Imaging" nachweislich (Messung in einem Magnetic Particle spectrometer) vergleichbar gutes Signalverhalten auf wie die derzeit als "Goldstandard" verwendeten Proben mit Resovist^{®} (Resovist^{®} enthält Ferucarbotran d.h. eine kolloide, wässrige Suspension superparamagnetischer Eisenoxid-Partikel [Mischung aus Magnetit Fe₃O₄ und Maghemit γ-Fe₂O₃], die von Carboxydextran umhüllt sind).

### Beispiel 2 - Herstellung von Einkern-Nanopartikel in im Wesentlichen sphärischer Form bzw. Quaderform

| | |
|---|---|
| Ausgangslösung BO: | 60 mM NaOH, 54 mM NaNO₃ in entgastem Wasser; |
| Ausgangslösung F: | 0,1 M FeCl₂ in entgastem Wasser; |
| Stabilisatorlösung S: | 20 mM Rutinhydrat in 60 mM NaOH. |

Alle Reaktionslösungen wurden auf 60 °C vorgeheizt, die Reaktion erfolgte bei 60 °C.

Pumpe A wurde mit 7 ml/min, Pumpe B mit 1,05 ml/min und Pumpe C mit 2, 1 ml/min betrieben. Die Verweilschleife mit Innendurchmesser 1/16" war in diesem Beispiel 50 m lang. Verwendet wurde ein Mikromischer mit der Bezeichnung "CPMM R300x12-SO" aus Polyetheretherketon. Die mit diesem Verfahren quaderförmigen Einkern-Nanopartikel sind in Fig. 2B dargestellt.

## Patentansprüche

1. Verfahren zur kontinuierlichen Herstellung von stabil dispergierbaren magnetischen Einkern-Nanopartikeln, die in wässrigen Medien nicht zur Aggregation neigen, die Eisenoxid enthalten und einen Durchmesser zwischen 20 und 200 nm aufweisen, umfassend die Schritte
a) Bereitstellen einer wässrigen Lösung enthaltend mindestens eine Base und mindestens ein Oxidationsmittel;
b) Bereitstellen einer wässrigen Lösung enthaltend mindestens ein Eisensalz, bevorzugt mindestens ein Fe(II)-Salz, besonders bevorzugt mindestens ein Fe(II)-Salz und ein von einem Fe(II)-Salz verschiedenes Eisensalz in einer niedrigeren Konzentration als das Fe(II)-Salz;
c) Bereitstellen einer wässrigen Lösung enthaltend mindestens einen hydrophilen Stabilisator, wobei der hydrophile Stabilisator eine Substanz enthält oder daraus besteht, die ausgewählt ist aus der Gruppe bestehend aus glykosidische Flavonoide, Phenole, Phosphorsäurederivate, Triphosphat, Polymere mit mehr als 3 Histidin-Resten, hydrophile Polymere und Carbonsäure oder Carbonsäureanhydrid;
d) Mischen der wässrigen Lösung aus a) und b) zu einem Gemisch in einem Mikromischer, wobei Fe(OH)₂ gebildet wird, das aus der Lösung ausfällt und zu magnetischen Einkern-Nanopartikeln oxidiert;
e) Mischen des Gemisches aus d) mit der wässrigen Lösung aus c), wobei der mindestens eine Stabilisator an das Eisenoxid bindet;
**dadurch gekennzeichnet, dass** in a) bis e) auf eine Temperatur von 10°C bis 200 °C temperiert wird, wobei in Schritt e) stabil dispergierbare magnetische Einkern-Nanopartikel entstehen, die in wässrigen Medien nicht zur Aggregation neigen, die Eisenoxid und den Stabilisator enthalten und einen Durchmesser zwischen 20 und 200 nm aufweisen.

2. Verfahren gemäß Anspruch 1, **dadurch gekennzeichnet, dass** in Schritt d), optional auch in Schritt e), mit einem Mikromischer gemäß DIN EN ISO 10991:2010-03 gemischt wird; und/oder der Mikromischer einen Multilaminations-Mikromischer, "split-and-recombine"-Mikromischer und/oder einen "impinging-jet"-Mikromischer enthält oder daraus besteht, wobei der Mikromischer optional im Anschluss an den Mischbereich oder Mischraum durch einen sich im Wesentlichen nicht verengenden und/oder im Wesentlichen geraden Ausgang ohne abrupte Richtungsänderung und/oder Querschnittsverengung des Fluidstroms gekennzeichnet ist.

3. Verfahren gemäß einem der vorhergehenden Ansprüche, **dadurch gekennzeichnet, dass** der hydrophile Stabilisator eine Substanz ausgewählt aus der Gruppe bestehend aus
a) Rutin und/oder Hesperidin, besonders bevorzugt Rutinhydrat;
b) Polyphenole, weiter bevorzugt Quercetin, Tannin, Catechol und/oder Lignin, besonders bevorzugt Ligninsulfonat und/oder Catechol-PEG;
c) Bisphosphonat, besonders bevorzugt Alendronsäure und Derivate hiervon;
d) Pentanatriumtriphosphat und/oder Pentakaliumtriphosphat;
e) Stärke, Chitosan, Dextran; und
f) Polymaleinsäureanhydrid und Derivate hiervon;
enthält oder daraus besteht, bevorzugt glykosidische Flavonoide und/oder hydrophile Polymere, besonders bevorzugt ein kovalent mit einem hydrophilen Polymer verknüpftes glykosidisches Flavonoid, insbesondere Rutin-PEG.

4. Verfahren gemäß einem der vorhergehenden Ansprüche, **dadurch gekennzeichnet, dass** die wässrige Lösung in a), b), und/oder c), bevorzugt a) bis c), in demineralisiertem und/oder entgastem Wasser bereitgestellt wird, insbesondere mit entgastem Wasser, das weniger als 50 %, vorzugweise weniger als 75%, des im Gleichgewicht bei 20 °C und Atmosphärendruck gelösten Sauerstoffs aufweist; und/oder in dem Schritt a), b), c), d) und/oder e) bevorzugt in den Schritten a) bis e), auf eine Temperatur von 10 °C bis 200 °C, bevorzugt 40 bis 100 °C, besonders bevorzugt 50 bis 70 °C, insbesondere 60 °C, temperiert wird.

5. Verfahren gemäß einem der vorhergehenden Ansprüche, **dadurch gekennzeichnet, dass** vor oder nach Zugabe des Stabilisators für einen Zeitraum von 1 sek. bis 24 h, bevorzugt 10 sek. bis 4 h, besonders bevorzugt 30 sek. bis 40 min., insbesondere 1 min. bis 20 min., inkubiert wird, insbesondere in einer temperierten Verweilschleife.

6. Verfahren gemäß einem der vorhergehenden Ansprüche, **dadurch gekennzeichnet, dass** in einem weiteren Schritt die Oberfläche der stabilisierten magnetischen Einkern-Nanopartikel modifiziert wird, wobei der hierbei genutzte Modifikator bevorzugt eine Substanz ausgewählt aus der Gruppe bestehend aus
a) anorganische Oxide, bevorzugt Siliciumoxid;
b) anorganische Phosphate, bevorzugt Hydroxyapatit;
c) Hartstoffe, bevorzugt Siliciumcarbid;
d) organische Polymere, bevorzugt PEG, PEG-Silan, Polylactid, Polystyrol, Polymethacrylat, Polyvinylalkohol, Polyvinylpyrrolidon, Polyglycolid und/oder Polycaprolacton; und/oder
e) anorganische Polymere, bevorzugt Polysiloxan;
enthält oder daraus besteht.

7. Verfahren gemäß einem der vorhergehenden Ansprüche, **dadurch gekennzeichnet, dass** die Konzentration
a) der Base 10 mM bis 5 M beträgt, bevorzugt 15 mM bis 1 M, besonders bevorzugt 20 mM bis 120 mM;
b) des Oxidationsmittels 10 mM bis 5 M beträgt, bevorzugt 15 mM bis 1 M, besonders bevorzugt 20 mM bis 120 mM; und/oder
c) des Fe(II)-Salzes 10 mM bis 5 M beträgt, bevorzugt 50 mM bis 500 mM, besonders bevorzugt 100 mM bis 200 mM; und/oder
d) des Stabilisators 1mM bis 5 M beträgt, bevorzugt 10 mM bis 1 M, besonders bevorzugt 15 mM bis 120 mM, insbesondere 20 mM bis 50 mM.

8. Verfahren gemäß einem der vorhergehenden Ansprüche, **dadurch gekennzeichnet, dass** das molare Konzentrationsverhältnis der Fe²⁺-Ionen zu den OH⁻-Ionen 4:1 bis 1:8, bevorzugt 2:1 bis 1:4, beträgt.

9. Verfahren gemäß einem der vorhergehenden Ansprüche, **dadurch gekennzeichnet, dass**
i) die Base ein Alkalihydroxid und/oder Ammoniumhydroxid enthält oder daraus besteht, bevorzugt Natriumhydroxid, Kaliumhydroxid, Ammoniumhydroxid und/oder Trimethylammoniumhydroxid; und/oder
ii) das Oxidationsmittel ein Nitratsalz und/oder HNO₃ enthält oder daraus besteht, bevorzugt ein Alkalinitrat und/oder Ammoniumnitrat, besonders bevorzugt Natriumnitrat, Kaliumnitrat und/oder Ammoniumnitrat, und insbesondere das Oxidationsmittel keine Fe(III)-Ionen enthält, wobei vorzugsweise das molare Konzentrationsverhältnis der NO₃⁻-Ionen zu den Fe²⁺-Ionen 20:1 bis 1:4, bevorzugt 10:1 bis 1:1 und/oder vorzugsweise das molare Konzentrationsverhältnis der NO₃⁻-Ionen zu den OH⁻-Ionen 1:5 bis 10:1, bevorzugt 1:2 bis 5:1 beträgt und/oder
iii) das Fe(II)-Salz ein Fe(II)-Halogenid und/oder Fe(II)-Sulfat, bevorzugt Fe(II)chlorid und/oder Fe(II)sulfat, enthält oder daraus besteht.

10. Verfahren gemäß einem der vorhergehenden Ansprüche, **dadurch gekennzeichnet, dass** die Einkern-Nanopartikel in einem weiteren Schritt nach Schritt e) aufgereinigt werden, bevorzugt über Diafiltration und/oder Ultrafiltration, wobei die Einkern-Nanopartikel insbesondere von Salzen und überschüssigem Stabilisator befreit werden.

11. Magnetische Einkern-Nanopartikel mit einem Durchmesser zwischen 20 und 200 nm, enthaltend Eisenoxid und mindestens einen hydrophilen Stabilisator, **dadurch gekennzeichnet, dass** sie durch das Verfahren gemäß einem der Ansprüche 1 bis 10 herstellbar sind, wobei der hydrophile Stabilisator eine Substanz ausgewählt aus der Gruppe bestehend aus glykosidische Flavonoide, Phenole, Bisphosphonat, Triphosphat und Polymere mit mehr als 3 Histidin-Resten enthält oder daraus besteht.

12. Magnetische Einkern-Nanopartikel gemäß Anspruch 11, **dadurch gekennzeichnet, dass** die Einkern-Nanopartikel die Form eines Plättchens aufweisen, bevorzugt ein Plättchen mit einem Durchmesser von 20 bis 200 nm und/oder einer Dicke von 2 bis 20 nm, besonders bevorzugt mit einem Durchmesser von 20 bis 40 nm und einer Dicke von 2 bis 10 nm.

13. Magnetische Einkern-Nanopartikel gemäß einem der Ansprüche 11 oder 12 zur Verwendung in der Medizin, bevorzugt zur Verwendung in einem Verfahren zur chirurgischen oder therapeutischen Behandlung des menschlichen oder tierischen Körpers, besonders bevorzugt
a) zur Magnetfeld-gesteuerten Freisetzung von einem Wirkstoff;
b) zum Magnetfeld-gesteuerten Targeting von einem Wirkstoff;
c) zur Behandlung einer Krankheit mit Magnet-Fluid-Hyperthermie; und/oder
d) als Kontrastmittel oder Tracer in der medizinischen Bildgebung, bevorzugt in der Magnetresonanztomographie und/oder dem "Magnetic Particle Imaging";
in einem Verfahren zur chirurgischen oder therapeutischen Behandlung des menschlichen oder tierischen Körpers, wobei es sich bei der Behandlung insbesondere um die Behandlung von Krebs handelt.

14. Magnetische Einkern-Nanopartikel gemäß einem der Ansprüche 11 oder 12 zur Verwendung in der Diagnostik, bevorzugt als Sensor in der Diagnostik, besonders bevorzugt als Sensor in einem Diagnostizierverfahren, das am menschlichen oder tierischen Körper vorgenommen wird.

15. Verwendung der Einkern-Nanopartikel gemäß einem der Ansprüche 11 oder 12 zur
a) Magnetfeld-gesteuerten Freisetzung von Substanzen;
b) Magnetfeld-gesteuerten Separation von Substanzen;
c) Magnetfeld-gesteuerten Schaltung elektronischer Komponenten;
d) Verwendung als Kontrastmittel oder Tracer in der Bildgebung, bevorzugt in der Magnetresonanztomographie und/oder dem "Magnetic Particle Imaging";
e) Herstellung von Kompositmaterialien; und/oder
f) Herstellung eines Ferrofluids.

## Claims

1. Method for continuous production of stably dispersible, magnetic, single-core nanoparticles which do not aggregate in aqueous media, comprise iron oxide and have a diameter between 20 and 200 nm, comprising the steps
a) preparing an aqueous solution comprising at least one base and at least one oxidant;
b) preparing an aqueous solution comprising at least one iron salt, preferably at least one Fe(II) salt, particularly preferably at least one Fe(II) salt and an iron salt which is different from an Fe(ll) salt in a lower concentration than the Fe(ll) salt;
c) preparing an aqueous solution comprising at least one hydrophilic stabiliser, wherein the hydrophilic stabiliser comprises or consists of a substance selected from the group consisting of glycosidic flavonoids, phenols, phosphoric acid derivatives, triphosphate, polymers with more than 3 histidine residues, hydrophilic polymers and carboxylic acid or carboxylic anhydride;
d) mixing the aqueous solution of a) and b) to form a mixture in a micromixer, wherein Fe(OH)₂ is formed which precipitates out of the solution and oxidises to form magnetic single-core nanoparticles;
e) mixing of the mixture from d) with the aqueous solution from c), wherein the at least one stabiliser binds to the iron oxide.
**characterized in that**, in a) to e), it is tempered to a temperature of 10°C to 200°C, wherein, in step e), stably dispersible, magnetic, single-core nanoparticles are formed which do not aggregate in aqueous media, comprise iron oxide and the stabiliser and have a diameter between 20 and 200 nm.

2. Method according to claim 1, **characterised in that**, in step d), optionally also in step e), mixing takes place with a micromixer according to DIN EN ISO 10991:2010-03; and/or the micromixer comprises or consists of a multilamination micromixer, "split-and-recombine" micromixer and/or an "impinging jet" micromixer, wherein the micromixer is optionally **characterized by**, following the mixing region or mixing chamber, an essentially non-tapering and/or essentially straight exit without abrupt change in direction and/or tapering in cross-section of the fluid flow.

3. Method according to one of the preceding claims, **characterised in that** the hydrophilic stabiliser comprises or consists of a substance selected from the group consisting of
a) rutin and/or hesperidin, particularly preferably rutin hydrate;
b) polyphenols, further preferably quercetin, tannin, catechol and/or lignin, particularly preferably lignin sulphonate and/or catechol-PEG;
c) bisphosphonate, particularly preferably alendronic acid and derivatives hereof;
d) pentasodium triphosphate and/or pentapotassium triphosphate;
e) starch, chitosan, dextran; and
f) polymaleic anhydride and derivatives hereof;
preferably glycosidic flavonoids and/or hydrophilic polymers, particularly preferably a glycosidic flavonoid crosslinked covalently with a hydrophilic polymer, in particular rutin-PEG.

4. Method according to one of the preceding claims, **characterised in that**
the aqueous solution in a), b), and/or c), preferably a) to c), is prepared in demineralised and/or degassed water, particularly in degassed water which has less than 50%, preferably less than 75%, of the oxygen dissolved in equilibrium at 20°C and at atmospheric pressure; and/or
in step a), b), c), d) and/or e), preferably in steps a) to e), it is tempered to a temperature of 10°C to 200°C, preferably 40 to 100°C, particularly preferably 50 to 70°C, in particular 60°C.

5. Method according to one of the preceding claims, **characterised in that** incubation takes place before or after addition of the stabiliser for a time period of 1 sec. to 24 h, preferably 10 sec. to 4 h, particularly preferably 30 sec. to 40 min., in particular 1 min. to 20 min, in particular in a temperature-controlled dwell loop.

6. Method according to one of the preceding claims, **characterised in that**, in a further step, the surface of the stabilised, magnetic, single-core nanoparticles is modified, wherein the hereby used modifier preferably comprises or consists of a substance selected from the group consisting of
a) inorganic oxides, preferably silicon oxide;
b) inorganic phosphates, preferably hydroxyapatite;
c) hard materials, preferably silicon carbide;
d) organic polymers, preferably PEG, PEG-silane, polylactide, polystyrene, polymethacrylate, polyvinyl alcohol, polyvinylpyrrolidone, polyglycolide and/or polycaprolactone; and/or
e) inorganic polymers, preferably polysiloxane.

7. Method according to one of the preceding claims, **characterised in that** the concentration
a) of the base is 10 mM to 5 M, preferably 15 mM to 1 M, particularly preferably 20 mM to 120 mM;
b) of the oxidant is 10 mM to 5 M, preferably 15 mM to 1 M, particularly preferably 20 mM to 120 mM; and/or
c) of the Fe(ll) salt is 10 mM bis 5 M, preferably 50 mM to 500 mM, particularly preferably 100 mM to 200 mM; and/or
d) of the stabiliser is 1 mM bis 5 M, preferably 10 mM to 1 M, particularly preferably 15 mM to 120 mM, in particular 20 mM to 50 mM.

8. Method according to one of the preceding claims, **characterised in that** the molar concentration ratio of the Fe²⁺ ions to the OH⁻ ions is 4:1 to 1:8, preferably 2:1 to 1:4.

9. Method according to one of the preceding claims, **characterised in that**
i) the base comprises or consists of an alkali hydroxide and/or ammonium hydroxide, preferably sodium hydroxide, potassium hydroxide, ammonium hydroxide and/or trimethylammonium hydroxide.
ii) the oxidant comprises or consists of a nitrate salt and/or HNO₃, preferably an alkali nitrate and/or ammonium nitrate, particularly preferably sodium nitrate, potassium nitrate and/or ammonium nitrate, and in particular, the oxidant comprises no Fe(III) ions, wherein particularly preferably the molar concentration ratio of the NO₃⁻ions to the Fe²⁺ ions is 20:1 to 1:4, preferably 10:1 to 1:1, and/or particularly preferably the molar concentration ratio of the NO₃⁻ions to the OH⁻ ions is 1:5 to 10:1, preferably 1:2 to 5:1; and/or
iii) the Fe(II) salt comprises or consists of an Fe(ll) halogenide and/or Fe(II) sulphate, preferably Fe(II) chloride and/or Fe(ll) sulphate.

10. Method according to one of the preceding claims, **characterised in that** the single-core nanoparticles are purified in a further step after step e), preferably via diafiltration and/or ultrafiltration, wherein the single-core nanoparticles are freed in particular of salts and excess stabiliser.

11. Magnetic, single-core nanoparticles with a diameter between 20 and 200 nm, comprising iron oxide and at least one hydrophilic stabiliser, **characterised in that** they are producible by the method according to one of the claims 1 to 10, wherein the hydrophilic stabiliser comprises or consists of a substance selected from the group consisting of glycosidic flavonoids, phenols, bisphosphonate, triphosphate and polymers with more than 3 histidine residues.

12. Magnetic, single-core nanoparticles according to claim 11, **characterised in that** the single-core nanoparticles have the shape of a platelet, preferably a platelet with a diameter of 20 to 200 nm and/or a thickness of 2 to 20 nm, particularly preferably with a diameter of 20 to 40 nm and/or a thickness of 2 to 10 nm.

13. Magnetic, single-core nanoparticles according to one of the claims 11 or 12 for use in medicine, preferably for use in a method for surgical or therapeutic treatment of the human or animal body, particularly preferably
a) for magnetic field-controlled release of an active substance;
b) for magnetic field-controlled targeting of an active substance;
c) for treatment of an illness with magnetic fluid hyperthermia; and/or
d) as contrast means or tracer in medical imaging, preferably in magnetic resonance tomography and/or magnetic particle imaging;
in a method for surgical or therapeutic treatment of the human or animal body, wherein the treatment is in particular the treatment of cancer.

14. Magnetic, single-core nanoparticles according to one of the claims 11 or 12 for use in diagnostics, preferably as sensor in diagnostics, particularly preferably as sensor in a diagnostics method which is carried out on the human or animal body.

15. Use of the single-core nanoparticles according to one of the claims 11 or 12 for
a) magnetic field-controlled release of substances;
b) magnetic field-controlled separation of substances;
c) magnetic field-controlled switching of electronic components;
d) use as contrast means or tracers in imaging, preferably in magnetic resonance tomography and/or in "magnetic particle imaging"
e) production of composite materials; and/or
f) production of a ferrofluid.

## Revendications

1. Procédé de fabrication en continu de nanoparticules magnétiques mononucléaires dispersables de manière stable, qui ne tendent pas à s'agréger dans des milieux aqueux, qui contiennent de l'oxyde de fer et présentent un diamètre compris entre 20 et 200 nm, comprenant les étapes de
a) préparation d'une solution aqueuse contenant au moins une base et au moins un agent oxydant ;
b) préparation d'une solution aqueuse contenant au moins un sel de fer, de préférence au moins un sel de Fe(II), de manière particulièrement préférée au moins un sel de Fe(II) et un sel de fer différent d'un sel de Fe(II) en une concentration inférieure au sel de Fe(II) ;
c) préparation d'une solution aqueuse contenant au moins un stabilisant hydrophile, dans lequel le stabilisant hydrophile contient une substance ou en est constitué, qui est choisie dans le groupe constitué par les flavonoïdes glycosidiques, les phénols, les dérivés de l'acide phosphorique, le triphosphate, les polymères avec plus de 3 résidus histidine, les polymères hydrophiles et l'acide carboxylique ou l'anhydride carboxylique ;
d) mélange de la solution aqueuse composée de a) et b) en un mélange dans un micromélangeur, dans lequel du Fe(OH)₂ est formé, qui se précipite à partir de la solution et s'oxyde en nanoparticules magnétiques mononucléaires ;
e) mélange du mélange composé de d) avec la solution aqueuse composée de c), dans lequel le au moins un stabilisant se lie à l'oxyde de fer ;
**caractérisé en ce qu'**une thermorégulation est effectuée dans a) à e) à une température de 10 °C à 200 °C, dans lequel des nanoparticules magnétiques mononucléaires dispersables de manière stable se forment dans l'étape e), qui ne tendent pas à s'agréger dans des milieux aqueux, qui contiennent de l'oxyde de fer et le stabilisant et présentent un diamètre compris entre 20 et 200 nm.

2. Procédé selon la revendication 1, **caractérisé en ce que** dans l'étape d), éventuellement également dans l'étape e), un mélange est effectué avec un micromélangeur selon DIN EN ISO 10991:2010-03 ; et/ou le micromélangeur contient un micromélangeur multicouche, un micromélangeur « sépare-et-recombine » et/ou un micromélangeur « à impact de jet » ou en est constitué, dans lequel le micromélangeur est éventuellement caractérisé, à la suite de la zone de mélange ou chambre de mélange, par une sortie sensiblement non rétrécie et/ou sensiblement droite sans changement brusque de direction et/ou rétrécissement de la section transversale du flux de fluide.

3. Procédé selon l'une quelconque des revendications précédentes, **caractérisé en ce que** le stabilisant hydrophile contient une substance choisie dans le groupe constitué par
a) la rutine et/ou l'hespéridine, de manière particulièrement préférée l'hydrate de rutine ;
b) les polyphénols, de préférence encore la quercétine, le tanin, le catéchol et/ou la lignine, de manière particulièrement préférée le lignosulfonate et/ou le catéchol-PEG ;
c) le bisphosphonate, de manière particulièrement préférée l'acide alendronique et ses dérivés ;
d) le triphosphate pentasodique et/ou triphosphate pentapotas-sique ;
e) l'amidon, le chitosan, le dextran ; et
f) l'anhydride polymaléique et ses dérivés ;
ou en est constitué, de préférence des flavonoïdes glycosidiques et/ou des polymères hydrophiles, de manière particulièrement préférée un flavonoïde glycosidique lié par covalence à un polymère hydrophile, en particulier la rutine-PEG.

4. Procédé selon l'une quelconque des revendications précédentes, **caractérisé en ce que** la solution aqueuse est préparée dans l'étape a), b), et/ou c), de préférence a) à c), dans de l'eau déminéralisée et/ou dégazée, notamment avec de l'eau dégazée, qui présente moins de 50 %, de préférence moins de 75 %, d'oxygène dissous à l'équilibre à 20 °C et à la pression atmosphérique ; et/ou dans l'étape a), b), c), d) et/ou e), de préférence dans les étapes a) à e), est thermorégulée à une température de 10 °C à 200 °C, de préférence de 40 à 100 °C, de manière particulièrement préférée de 50 à 70 °C, en particulier de 60 °C.

5. Procédé selon l'une quelconque des revendications précédentes, **caractérisé en ce qu'**avant ou après l'addition du stabilisant, une incubation est effectuée pendant une durée de 1 seconde à 24 heures, de préférence de 10 secondes à 4 heures, de manière particulièrement préférée de 30 secondes à 40 minutes, en particulier de 1 minute à 20 minutes, notamment dans une boucle de séjour thermorégulée.

6. Procédé selon l'une quelconque des revendications précédentes, **caractérisé en ce que**, dans une étape supplémentaire, la surface des nanoparticules magnétiques mononucléaires stabilisées est modifiée, dans lequel le modificateur utilisé à cet effet contient de préférence une substance choisie dans le groupe constitué par
a) les oxydes inorganiques, de préférence l'oxyde de silicium ;
b) les phosphates inorganiques, de préférence l'hydroxyapatite ;
c) les matériaux durs, de préférence le carbure de silicium ;
d) les polymères organiques, de préférence les PEG, PEG-silane, polylactide, polystyrène, polyméthacrylate, alcool polyvinylique, polyvinylpyrrolidone, polyglycolide et/ou polycaprolactone ; et/ou
e) les polymères inorganiques, de préférence le polysiloxane ; ou en est constitué.

7. Procédé selon l'une quelconque des revendications précédentes, **caractérisé en ce que** la concentration
a) de la base est de 10 mM à 5 M, de préférence de 15 mM à 1 M, de manière particulièrement préférée de 20 mM à 120 mM ;
b) de l'oxydant est de 10 mM à 5 M, de préférence de 15 mM à 1 M, de manière particulièrement préférée de 20 mM à 120 mM ; et/ou
c) du sel de Fe(II) est de 10 mM à 5 M, de préférence de 50 mM à 500 mM, de manière particulièrement préférée de 100 mM à 200 mM ; et/ou
d) du stabilisant est de 1 mM à 5 M, de préférence de 10 mM à 1 M, de manière particulièrement préférée de 15 mM à 120 mM, en particulier de 20 mM à 50 mM.

8. Procédé selon l'une quelconque des revendications précédentes, **caractérisé en ce que** le rapport de concentration molaire des ions Fe²⁺ sur les ions OH⁻ est 4:1 à 1:8, de préférence 2:1 à 1:4.

9. Procédé selon l'une quelconque des revendications précédentes, **caractérisé en ce que**
i.) la base contient un hydroxyde alcalin et/ou hydroxyde d'ammonium ou en est constituée, de préférence l'hydroxyde de sodium, l'hydroxyde de potassium, l'hydroxyde d'ammonium et/ou l'hydroxyde de triméthylammonium ; et/ou
ii.) l'oxydant contient un sel de nitrate et/ou HNO₃ ou en est constitué, de préférence un nitrate alcalin et/ou nitrate d'ammonium, de manière particulièrement préférée nitrate de sodium, nitrate de potassium et/ou nitrate d'ammonium, et en particulier l'oxydant ne contient pas d'ions Fe(III), dans lequel de préférence le rapport de concentration molaire des ions NO₃⁻ aux ions Fe²⁺ est 20:1 à 1:4, de préférence 10:1 à 1:1 et/ou de préférence le rapport de concentration molaire des ions NO₃⁻ aux ions OH⁻ est 1:5 à 10:1, de préférence 1:2 à 5:1 et/ou
iii.) le sel de Fe(II) contient un halogénure de Fe(II) et/ou sulfate de Fe(II), de préférence chlorure de Fe(II) et/ou sulfate de Fe(II), ou en est constitué.

10. Procédé selon l'une quelconque des revendications précédentes, **caractérisé en ce que** les nanoparticules mononucléaires sont purifiées dans une étape supplémentaire après l'étape e), de préférence par diafiltration et/ou ultrafiltration, dans lequel les nanoparticules mononucléaires sont notamment débarrassées des sels et du stabilisant en excès.

11. Nanoparticules magnétiques mononucléaires avec un diamètre compris entre 20 et 200 nm, contenant de l'oxyde de fer et au moins un stabilisant hydrophile, **caractérisées en ce qu'**elles peuvent être obtenues par le procédé selon l'une quelconque des revendications 1 à 10, dans lequel le stabilisant hydrophile contient une substance choisie dans le groupe constitué par les flavonoïdes glycosidiques, les phénols, le bisphosphonate, le triphosphate et les polymères avec plus de 3 résidus histidine ou en est constitué.

12. Nanoparticules magnétiques mononucléaires selon la revendication 11, **caractérisées en ce que** les nanoparticules mononucléaires présentent la forme d'une plaquette, de préférence une plaquette avec un diamètre de 20 à 200 nm et/ou une épaisseur de 2 à 20 nm, de manière particulièrement préférée avec un diamètre de 20 à 40 nm et une épaisseur de 2 à 10 nm.

13. Nanoparticules magnétiques mononucléaires selon l'une quelconque des revendications 11 ou 12 pour leur utilisation en médecine, de préférence pour leur utilisation dans un procédé de traitement chirurgical ou thérapeutique du corps humain ou animal, de manière particulièrement préférée
a) pour la libération commandée par champ magnétique d'une substance active ;
b) pour le ciblage commandé par champ magnétique d'une substance active ;
c) pour le traitement d'une maladie par hyperthermie par fluide magnétique ; et/ou
d) comme agent de contraste ou traceur en imagerie médicale, de préférence en imagerie par résonance magnétique et/ou en « imagerie à particules magnétiques » ;
dans un procédé de traitement chirurgical ou thérapeutique du corps humain ou animal, dans lequel le traitement est notamment le traitement du cancer.

14. Nanoparticules magnétiques mononucléaires selon l'une quelconque des revendications 11 ou 12 pour leur utilisation en diagnostic, de préférence en tant que capteur en diagnostic, de manière particulièrement préférée en tant que capteur dans un procédé de diagnostic qui est réalisé sur le corps humain ou animal.

15. Utilisation des nanoparticules mononucléaires selon l'une quelconque des revendications 11 ou 12 pour
a) la libération de substances commandée par champ magnétique ;
b) la séparation de substances commandée par champ magnétique ;
c) la commutation de composants électroniques commandée par champ magnétique ;
d) l'utilisation comme agent de contraste ou traceur en imagerie, de préférence en imagerie par résonance magnétique et/ou en « imagerie à particules magnétiques » ;
e) la fabrication de matériaux composites ; et/ou
f) l'obtention d'un ferrofluide.
